# EUROPÄISCHE PATENTSCHRIFT

(11) **EP 1 633 807 B1**
(45) Veröffentlichungstag und Bekanntmachung des Hinweises auf die Patenterteilung: **21.12.2016**
(21) Anmeldenummer: 04739671.8
(22) Anmeldetag: 07.06.2004
(51) Int. Cl.: C08J 9/26, A61L 27/18, A61L 27/26, A61L 27/38, A61L 27/56, A61L 27/58

(54) **MATRIX, ZELLIMPLANTAT, VERFAHREN ZU DEREN HERSTELLUNG UND DEREN VERWENDUNG**
MATRIX, CELL IMPLANTATION AND METHOD FOR THEIR PRODUCTION AND USE
MATRICE, IMPLANT CELLULAIRE, LEUR PROCEDE DE PRODUCTION ET LEUR UTILISATION

(30) Priorität: 06.06.2003 DE 10325807
(43) Veröffentlichungstag der Anmeldung: 15.03.2006
(60) Teilanmeldung: 10179005.3
(73) Patentinhaber: Humanautocell GmbH, 80802 München (DE)
(72) Erfinder: GÖRNE, Martin, 22337 Hamburg (DE); KAUFMANN, Peter, Matthias, 30900 Wedemark (DE)
(74) Vertreter: Reitstötter Kinzebach
(86) Internationale Anmeldenummer: PCT/EP2004/006140
(87) Internationale Veröffentlichungsnummer: WO 2004/108810

(56) Entgegenhaltungen:
- WO-A-01/35932
- WO-A-98/44027
- WO-A-99/09149
- WO-A-99/32204
- WO-A-03/064509
- US-A- 5 626 861
- US-B1- 6 337 198
- HOU Q ET AL: "Porous polymeric structures for tissue engineering prepared by a coagulation, compression moulding and salt leaching technique" BIOMATERIALS, ELSEVIER SCIENCE PUBLISHERS BV., BARKING, GB, Bd. 24, Nr. 11, Mai 2003 (2003-05), Seiten 1937-1947, XP004412414 ISSN: 0142-9612
- SOHIER J ET AL: "A novel method to obtain protein release from porous polymer scaffolds: emulsion coating" JOURNAL OF CONTROLLED RELEASE, ELSEVIER SCIENCE PUBLISHERS B.V. AMSTERDAM, NL, Bd. 87, Nr. 1-3, 21. Februar 2003 (2003-02-21), Seiten 57-68, XP004412747 ISSN: 0168-3659
- CHEN G ET AL: "Preparation of poly(l-lactic acid) and poly(dl-lactic-co-glycolic acid) foams by use of ice microparticulates" BIOMATERIALS, ELSEVIER SCIENCE PUBLISHERS BV., BARKING, GB, Bd. 22, Nr. 18, September 2001 (2001-09), Seiten 2563-2567, XP004273584 ISSN: 0142-9612
- HARRIS L D ET AL: "OPEN PORE BIODEGRADABLE MATRICES FORMED WITH GAS FOAMING" JOURNAL OF BIOMEDICAL MATERIALS RESEARCH, WILEY, NEW YORK, NY, US, Bd. 42, 1998, Seiten 396-402, XP001021110 ISSN: 0021-9304
- WHANG K ET AL: "A novel method to fabricate bioabsorbable scaffolds" POLYMER, ELSEVIER SCIENCE PUBLISHERS B.V, GB, Bd. 36, Nr. 4, 1. Februar 1995 (1995-02-01), Seiten 837-842, XP004025968 ISSN: 0032-3861

## Beschreibung

Die vorliegende Erfindung betrifft poröse Matrices auf Basis eines biologisch verträglichen Polymers oder Polymergemisches, darauf aufbauende Zellimplantate, weitere Zeilimplantate auf Basis von Zellgemischen aus Hepatozyten und Langerhans'schen Inselzellen, ein Verfahren zur Herstellung poröser Matrices sowie die nach diesem Verfahren erhältlichen Matrices, und ein spezielles Verfahren zur Gewinnung von Zellen für die Beimpfung einer implantierbaren Matrix.

Tissue engineering ist ein interdisziplinäres Gebiet, das Ingenieur- und Materialwissenschaften mit der Medizin verbindet. Ziel ist es, geschädigtes Gewebe wiederherzustellen oder seine Funktion zu verbessern.

Das Prinzip des Tissue engineering ist denkbar einfach: Zunächst werden dem Patienten einige Zellen entnommen und *in vitro* vermehrt. Die vermehrten Zellen können dann in eine Gerüstsubstanz eingebettet werden, wodurch ein kompletter, lebender Gewebeersatz entsteht, der dem Patienten wieder transplantiert wird. Im Gegensatz zur herkömmlichen, einen geeigneten Spender voraussetzenden und in der Regel eine lebenslängliche medikamentöse Immunsuppression erfordernden allogenen Transplantation bietet dieses Verfahren den entscheidenden Vorteil, körpereigene (autologe) Zellen verwenden zu können.

Von besonderer Bedeutung für die Annahme und Funktionsfähigkeit der Implantate sind Art und Aufbau der verwendeten Gerüstsubstanz, im folgenden auch Matrix genannt. Abgesehen von dem zu verwendenden Material, nämlich in der Regel biologisch abbaubaren Polymeren, spielen Porengröße, Porosität und Oberfläche genauso wie die Porengestalt, die Morphologie der Porenwand und die Konnektivität zwischen den Poren eine entscheidende Rolle für die weitere Entwicklung der in der Gerüstsubstanz eingebetteten Zellen und letztendlich für den dreidimensionalen Aufbau des zu regenerierenden Gewebes oder Organs.

Verfahren zur Erzeugung derartiger Biomatrices sind bereits bekannt. So wurden bereits Techniken aus dem Textilbereich angewendet, um webartige und auch vliesartige, faserige Biomatrices herzustellen. Ein weiteres geläufiges Verfahren, bei dem Salzkristalle zunächst in das biologisch abbaubare Polymer eingearbeitet und anschließend wieder herausgelöst werden, ermöglicht es, die Porengröße über die Größe der Salzpartikel und die Porosität über das Salz/Polymerverhältnis zu kontrollieren (WO 98/44027). Bei einer Abwandlung des Verfahrens werden die in einem Lösungsmittel gelösten, biologisch abbaubaren Polymere auf ein sogenanntes porogenes Material aufgetragen, das anschließend aus dem Verbundmaterial wieder herausgelöst wird, wodurch Poren mit der Gestalt des negativen Abbildes besagten porogenen Materials hinterlassen werden (WO 01/87575 A2). Auch beschichtete Matrices sind bereits bekannt (siehe z.B. WO 99/09149 A1). Hou und Mitarbeiter beschreiben in Biomaterials, Bd. 24, Seiten 1937-1947 eine Reihe verschiedener Verfahren zur Herstellung polymerer Matrices. WO 01/35932 beschreibt poröse Matrices auf Basis eines biologisch verträglichen Polymers und Verfahren zu deren Herstellung. WO 98/44027 beschreibt ein Verfahren zur Herstellung offenporiger, biologisch abbaubarer Matrices. US 6337198 B1 beschreibt bioabbaubare und biokompatible poröse Matrices mit einer im Wesentlichen kontinuierlichen Polymerphase.

Nichtsdestotrotz stellen die bisher mit diesem Verfahren erzeugten Biomatrices insbesondere im Hinblick auf die Annahme und Funktionsfähigkeit darauf aufbauender Implantate nicht in allen Fällen zufrieden. Insbesondere mit Leber- und Pankreasimplantaten ist bisher noch kein annehmbarer Ersatz der Organe gelungen.

Die der vorliegenden Erfindung zugrunde liegende Aufgabe, ein solches funktionsfähiges Implantat zur Verfügung zu stellen, löst die Erfindung durch bestimmte Biomatrices und entsprechende Implantate, die mit einem speziellen Verfahren erhältlich sind.

Gegenstand der vorliegenden Erfindung sind daher die in den Patentansprüchen definierten Gegenstände.

Der Porositätsgrad ist die zahlenmäßige Angabe in % zum Anteil des Porenvolumens am Gesamtvolumen der Matrix.

Mit Poren bezeichnet man die in der erfindungsgemäßen Matrix vorhandenen Hohlräume, die im vorliegenden Fall im 2-dimensionalen Schnitt eine eckige, insbesondere oktogonale bzw. 3-dimensional gesehen eine kantige Gestalt haben. Vorzugsweise ist die Gestalt des weiteren durch Ausziehungen gekennzeichnet, so dass man die Gestalt der Hohlräume mit der Form von Nervenzellen vergleichen kann. Die Größe einer Pore kann mit Hilfe eines Durchmessers angegeben werden, das heißt dem Mittel aus dem längsten und dem kürzesten Durchmesser der im 2-dimensionalen Schnitt erkennbaren Poren.

Eine erfindungsgemäße Matrix weist Poren mit unterschiedlichen Größen auf, wobei die Größen über einen bestimmten Bereich verteilt sind (Porengrößenverteilung). Erfindungsgemäß von Bedeutung ist, dass eine Matrix eine breite Porengrößenverteilung aufweist. Von besonderem Vorteil sind Matrices, die Poren mit einer Größe von 130 µm oder weniger aufweisen. Von besonderem Vorteil sind Matrices, die Poren mit einer Größe von 370 µm oder mehr aufweisen. Matrices, die sowohl Poren mit einer Größe 130 µm oder weniger als auch Poren mit einer Größe von 370 µm oder mehr aufweisen, gehören zur Erfindung. Diese Werte lassen sich beliebig zu Mindestbereichen, über die sich die Porengrößenverteilung erstrecken soll, kombinieren, wobei insbesondere die Bereiche 150 bis 300, 140 bis 350 und 130 bis 370 µm zu nennen sind. Insbesondere bevorzugt ist es, wenn die jeweilige Porengrößenverteilung Häufigkeitsmaxima außerhalb des Bereichs von 150 bis 300 µm aufweist, d.h. ein Häufigkeitsmaximum oberhalb einer Porengröße von 300 µm und ein weiteres Häufigkeitsmaximum unterhalb einer Porengröße von 150 µm liegt.

Eine typische erfindungsgemäße Matrix weist folgende Porengrößenverteilung auf. 0,5 % bis 6 %, vorzugsweise 1 % bis 5 %, noch bevorzugter 2 % bis 4 % und insbesondere 3 % Poren mit einem mittleren Durchmesser im Bereich von 70 bis 100 µm; 2 % bis 8 %, vorzugsweise 3 % bis 7 %, noch bevorzugter 4 % bis 6 % und insbesondere 5 % Poren mit einem mittleren Durchmesser im Bereich von 101 bis 115 µm; 2 % bis 8 %, vorzugsweise 3 % bis 7 %, noch bevorzugter 4 % bis 6 % und insbesondere 5 % Poren mit einem mittleren Durchmesser im Bereich von 116 bis 130 µm; 1 % bis 7 %, vorzugsweise 2 % bis 6 %, noch bevorzugter 3 % bis 5 % und insbesondere 4 % Poren mit einem mittleren Durchmesser im Bereich von 131 bis 300 µm; 11 % bis 23 %, vorzugsweise 13 % bis 21 %, noch bevorzugter 15 % bis 19 % und insbesondere 17 % Poren mit einem mittleren Durchmesser im Bereich von 301 bis 330 µm; 4 % bis 10 %, vorzugsweise 5 % bis 9 %, noch bevorzugter 6 % bis 8 % und insbesondere 7 % Poren mit einem mittleren Durchmesser im Bereich von 331 bis 360 µm; 5 % bis 17 %, vorzugsweise 7 % bis 15 %, noch bevorzugter 9 % bis 13 % und insbesondere 11 % Poren mit einem mittleren Durchmesser im Bereich von 361 bis 390 µm; 7 % bis 19 %, vorzugsweise 9 % bis 17 %, noch bevorzugter 11 % bis 15 % und insbesondere 13 % Poren mit einem mittleren Durchmesser im Bereich von 391 bis 420 µm; 3 % bis 9 %, vorzugsweise 4 % bis 8 %, noch bevorzugter 5 % bis 7 % und insbesondere 6 % Poren mit einem mittleren Durchmesser im Bereich von 421 bis 450 µm; 12 % bis 24 %, vorzugsweise 14 % bis 22 %, noch bevorzugter 16 % bis 20 % und insbesondere 18 % Poren mit einem mittleren Durchmesser im Bereich von 451 bis 480 µm; und 5 % bis 17 %, vorzugsweise 7 % bis 15 %, noch bevorzugter 9 % bis 13 % und insbesondere 11 % Poren mit einem mittleren Durchmesser im Bereich von 481 bis 510 µm. Es ergibt sich also in der Regel eine Porengrößenverteilung mit mehr als einem Maximum, was einer Häufung von Poren in mehr als einem Größenbereich entspricht. Dies ist für die Eigenschaften erfindungsgemäßer Matrices von besonderer Bedeutung.

Das Hohlraumvolumen und damit der Porositätsgrad sind in an sich bekannter Weise durch Porosimetrie zu bestimmen.

Die Porengrößen und damit auch die Porengrößenverteilung können beispielsweise durch Rasterelektronenmikroskopie bestimmt werden. Dazu werden dünne Schnitte der zu untersuchenden Matrix angefertigt und mit Gold überzogen. Die rasterelektronenmikroskopischen Aufnahmen werden ausgewertet, indem man sämtliche Poren einer definierten Fläche ausmisst, d.h. für jede Pore den längsten und den kürzesten Durchmesser bestimmt, aus beiden Werten die Summe bildet und die Summe durch 2 dividiert.

Der Begriff "Matrix" bezieht sich auf einen dreidimensionalen Träger, der für die Ansiedlung von Zellen geeignet ist. In diesem Sinne dient die Matrix als dreidimensionale Strukturvorlage (Templat) für die Ansiedlung von Zellen bzw. Geweben. Diese Ansiedlung kann in vitro oder in vivo erfolgen. Ferner dient die Matrix bei Transplantationen zur Lokalisierung des Transplantats und auch als Platzhalter für Gewebe, das sich nach und nach in vivo bildet.

Das Polymer kann im Prinzip jedes im Bereich der Medizin und insbesondere der Transplantationsmedizin verwendbare Polymer sein. Biologisch verträglich sind demnach auch Polymere, die von einem Wirt zwar als fremd erkannt werden, deren Abstoßung sich aber durch entsprechende Immunsuppression unterdrücken lässt. Es können Polymere zum Einsatz kommen, die im Wesentlichen biologisch nicht abbaubar sind. Bevorzugt sind allerdings Polymere, die zumindest zum überwiegenden Teil biologisch abbaubar sind.

Der Ausdruck "biologisch abbaubar" bezieht sich auf ein Material, das Lebewesen (oder von Lebewesen ableitbare Körperflüssigkeiten oder Zellkulturen) in verstoffwechselbare Produkte zu überführen vermögen. Zu biologisch abbaubaren Polymeren gehören beispielsweise bioresorbierbare und/oder bioerodierbare Polymere. Bioerodierbar bezeichnet die Fähigkeit, in biologischen Flüssigkeiten lösbar oder suspendierbar zu sein. Bioresorbierbar meint die Fähigkeit, von Zellen, Gewebe oder Flüssigkeiten eines Lebewesens aufgenommen werden zu können.

Zur erfindungsgemäß geeigneten biologisch abbaubaren Polymeren gehören im Prinzip sämtliche im Bereich der Medizin verwendbaren Polymere, wozu neben den im Bereich des Tissue engineering bereits etablierten Polymeren beispielsweise auch Polymere gehören, die in Wirkstoffabgabevorrichtungen, wie Pflastern und Wirkstoffimplantaten, Eingang gefunden haben.

Zu geeigneten natürlichen Polymeren gehören beispielsweise Polypeptide wie Albumin, Fibrinogen, Collagen und Gelatine, sowie Polysaccharide, wie Chitin, Chitosan, Alginat und Agarose. Unter Umständen können diese natürlichen Polymere auch modifiziert sein, beispielsweise können Proteine wie Collagen quervernetzt sein.

Zu geeigneten synthetischen Polymeren gehören beispielsweise bestimmte Polyanhydride, insbesondere Poly(sebacinsäure-hexadecandisäure), Poly(ε-caprolacton), Poly(orthoester), und vor allem Poly(α-hydroxyester) wie Poly(glykolsäure), Poly(milchsäure) und Poly(glykolsäuremilchsäure). So basieren die erfindungsgemäßen Matrices und Implantate vorzugsweise auf biologisch abbaubaren Polymeren, die Wiederholungseinheiten der Formel (I) enthalten: 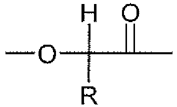 worin R¹ für Wasserstoff oder Methyl steht. Was die Milchsäureeinheiten angeht, so wird die L-Form (das S-Enantiomer) bevorzugt. Als besonders bevorzugtes Polymer ist Poly(glykolsäuremilchsäure) mit einem Glykolsäure zu Milchsäure-Verhältnis von 99:1 bis 1:99, vorzugsweise 10:90 bis 90:10, beispielsweise 15:85 mol-% zu nennen.

Gemische aus zwei oder mehreren Polymeren können ebenfalls zweckmäßig sein.

Neben der Art des Polymers bestimmt auch sein Molekulargewicht die Eigenschaften der resultierenden Matrix mit. Allgemein gilt, dass die Porosität der Matrix mit zunehmendem Molekulargewicht des verwendeten Polymers abnimmt. Dies gilt insbesondere dann, wenn bei der Herstellung der Matrix das Material geschäumt wird, d.h. unter Druck mit einem Gas wie CO₂ versetzt wird, das sich zunächst in dem Polymer löst und bei Absenkung des Drucks Poren bildet.

Ferner wirkt sich die Kristallinität des eingesetzten Polymers auf die Eigenschaften der resultierenden Matrix aus. Hier gilt, dass die Porosität der resultierenden Matrix mit abnehmender Kristallinität im allgemeinen zunimmt, weshalb amorphes Polymer insbesondere für Matrices mit hoher Porosität bevorzugt ist. Auch dieser Aspekt spielt insbesondere dann eine Rolle, wenn das Material bei der Herstellung der Matrix geschäumt wird.

Ein weiterer Gegenstand der vorliegenden Erfindung sind poröse Matrices auf Basis eines biologisch abbaubaren Polymers, die dadurch gekennzeichnet sind, dass die Oberfläche der Matrix mit wenigstens einem extrazellulären Matrixprotein beschichtet ist.

Extrazelluläre Matrixproteine sind allgemein bekannt. Erfindungsgemäß bevorzugt sind Collagene, insbesonders Collagene des Typs I und IV, Laminin und Fibronectin. Diese Proteine können in an sich bekannter Weise in aufgereinigter Form hergestellt oder auch kommerziell erworben werden. Gemäß einer Ausführungsform enthalten Beschichtungen erfindungsgemäßer Matrices als extrazelluläres Matrixprotein Fibronectin. Gemäß einer weiteren Ausführungsform enthalten Beschichtungen erfindungsgemäßer Matrices als extrazelluläres Matrixprotein ein Gemisch aus Collagen vom Typ I, Laminin und Collagen vom Typ IV, wobei es in diesem Fall bevorzugt ist, dass das Gemisch die Proteine in etwa gleichen Gewichtsanteilen enthält.

Erfindungsgemäß besonders bevorzugt sind Matrices, die in der oben beschriebenen Art und Weise beschichtet sind und die wenigstens eines der folgenden zusätzlichen Kriterien erfüllen:
- Die Poren der Matrices weisen die oben angegebenen Porengrößen bzw. Porengrößenverteilung auf;
- der Porositätsgrad beträgt 93 bis 98 %;
- die Poren weisen die oben angegebene Gestalt auf:
- das biologisch abbaubare Polymer ist eines der oben angegebenen natürlichen oder synthetischen Polymere, insbesondere Poly(glycolsäuremilchsäure) mit einem Milchsäureanteil von etwa 85 mol-% und einem Glykolsäureanteil von etwa 15 mol-%.

Derart beschichtete Matrices sind beispielsweise dadurch erhältlich, dass man die unbeschichtete Matrix in eine Lösung taucht, die das für die Beschichtung vorgesehene Protein oder Proteingemisch enthält, und anschließend die mit der Lösung befeuchtete Matrix trocknet. Dabei ist es in der Regel so, dass in Abhängigkeit von den Abmessungen des zu beschichtenden Matrixkörpers die Lösung vor allem die äußeren Bereiche des Matrixkörpers benetzt, während in das Innere des Matrixkörpers vergleichsweise weniger Lösung vordringt. Dies kann zur Folge haben, dass eine gleichmäßige Beschichtung der gesamten Matrixoberfläche nicht resultiert, sondern die Beschichtungsdichte von außen nach innen abnimmt.

Alternativ oder zusätzlich zu einer Beschichtung können biologisch aktive Substanzen im Polymer aufgenommen oder sogar damit verknüpft sein. Hierzu gehören beispielsweise synthetische Wirkstoffe (anorganische oder organische Moleküle), Proteine, Polysaccharide und weitere Zucker, Lipide und Nukleinsäuren, welche z.B. das Zellwachstum, die Zellmigration, die Zellteilung, die Zelldifferenzierung und/oder das Gewebewachstum beeinflussen, bzw. therapeutische, prophylaktische oder diagnostische Wirkungen besitzen. Zu nennen sind beispielsweise gefäßaktive Wirkstoffe, neuroaktive Wirkstoffe, Hormone, Wachstumsfaktoren, Cytokine, Steroide, Antikoagulanzien, entzündungshemmende Wirkstoffe, immunmodulierende Wirkstoffe, zytotoxische Wirkstoffe, Antibiotika und antivirale Wirkstoffe.

Die vorliegende Erfindung betrifft auch ein Verfahren zur Herstellung einer porösen Matrix auf Basis eines biologisch verträglichen Polymers oder Polymergemisches, das dadurch gekennzeichnet ist, dass man ein Gemisch aus Polymerpartikeln und Kochsalzpartikeln mit definierter Korngröße kompaktiert und anschließend das Kochsalz herauslöst.

Es hat sich ein Gewichtsverhältnis von Polymerpartikeln zu Kochsalzpartikeln im Bereich von 1:100 bis 1:10, vorteilhafterweise im Bereich von 1:50 bis 1:15 und insbesondere im Bereich von 1:20 bis 1:18 zur Einstellung der gewünschten Porosität als zweckmäßig erwiesen.

Es hat sich weiterhin als zweckmäßig erwiesen, Salz und Polymer mit einer bestimmten Korngrößenverteilung zu verwenden. Was das zur Herstellung der Matrix verwendete Kochsalz angeht, so ist es günstig, wenn der Anteil an Salz mit einer Korngröße von 250 µm bis 320 µm 22 % bis 28 %; der Anteil an Salz mit einer Korngröße von 330 µm bis 380 µm 42 % bis 46 %; und der Anteil an Salz mit einer Korngröße von 390 µm bis 425 µm 29 % bis 33 % beträgt, wobei sich die Prozentangaben auf das zur Herstellung insgesamt verwendete Gewicht an Salz beziehen. Anteile mit Korngrößen ober- und/oder unterhalb der angegebenen Bereiche sind damit nicht ausgeschlossen.

Es sich als günstig erwiesen, wenn der Anteil an Kochsalzpartikeln mit einer Korngröße von 108 µm bis 140 µm 7 bis 9 Gew.-%, der Anteil an Salz mit einer Korngröße von 145 µm bis 180 µm 5 bis 7 Gew.-%, der Anteil an Salz mit einer Korngröße von 185 µm bis 220 µm 10 bis 14 Gew.-%, der Anteil an Salz mit einer Korngröße von 225 µm bis 250 µm 5 bis 7 Gew.-%, der Anteil an Salz mit einer Korngröße von 250 µm bis 320 µm 22 bis 28 Gew.-%, der Anteil an Salz mit einer Korngröße von 330 µm bis 380 µm 22 bis 28 Gew.-%, und der Anteil an Salz mit einer Korngröße von 390 µm bis 425 µm 15 bis 19 Gew.-% beträgt.

Was das zur Herstellung der Matrix verwendete Polymer angeht, so ist es günstig, wenn der Anteil an Polymer mit einer Korngröße von 108 µm bis 140 pm 14 % bis 18 %; der Anteil an Polymer mit einer Korngröße von 145 pm bis 180 µm 20 % bis 24; der Anteil an Polymer mit einer Korngröße von 185 µm bis 220 µm 43 % bis 49 %, und der Anteil an Polymer mit einer Korngröße von 225 µm bis 250 µm 14 % bis 18 % beträgt, wobei sich die Prozentangaben auf das zur Herstellung insgesamt verwendete Gewicht an Polymer beziehen.

Um Salz- bzw. Polymerpartikel der gewünschten Korngrößenverteilung zu erhalten, ist es in der Regel zweckmäßig, handelsübliche Ware zunächst zu zerkleinern. Dies kann in dafür gebräuchlichen Vorrichtungen, z.B. Schlagwerken oder Mühlen, erfolgen. Bestimmend für die gewünschte Korngrößenverteilung ist allerdings das anschließende Aussieben mit Hilfe gebräuchlicher Analysesiebe.

Das Kompaktieren erfolgt vorzugsweise durch Einwirkung von Druck. Dazu kann man das Polymer/Kochsalz-Gemisch in einer herkömmlichen Hydraulikpresse bei einem Stempeldruck im Bereich von etwa 780 psi bis 1450 psi, vorteilhafterweise im Bereich von etwa 840 psi bis 1230 psi und insbesondere im Bereich von etwa 900 psi bis 1100 psi verpressen. Es hat sich als zweckmäßig erwiesen, den Druck etwa 10 s bis 360 s, vorteilhafterweise etwa 40 s bis 180 s und insbesondere etwa 50 s bis 70 s bei Temperaturen im Bereich von 18 °C bis 25 °C einwirken zu lassen.

Das Herauslösen des Kochsalzes gelingt beispielsweise mit Wasser oder wässrigen Lösungen. So kann man das kompaktierte Gemisch (Matrixrohling) etwa 1 h bis 80 h, vorteilhafterweise etwa 12 h bis 62 h und insbesondere etwa 36 h bis 60 h wässern.

Zudem ist es von Vorteil, wenn das kompaktierte Gemisch vor dem Herauslösen des Kochsalzes zunächst in einer CO₂-Atmosphäre gelagert wird. So kann man das kompaktierte Gemisch beispielsweise bei einem CO₂-Druck im Bereich von etwa 140 psi bis 1650 psi, vorteilhafterweise im Bereich von etwa 360 psi bis 1120 psi und insbesondere im Bereich von etwa 800 psi bis 900 psi begasen, wobei sich hierbei Zeiten im Bereich von etwa 1 h bis 180 h, vorteilhafterweise im Bereich von etwa 3 h bis 60 h und insbesondere im Bereich von etwa 12 h bis 36 h als zweckmäßig erwiesen haben. Danach verringert man den Druck, wobei die Druckabsenkungsgeschwindigkeit Einfluss auf die Porenbildung hat. Wenngleich die Verwendung von CO₂ bevorzugt ist, können andere Gase, wie Luft, Stickstoff, Helium, Neon, Krypton, Argon, Xenon oder Sauerstoff ebenfalls geeignet sein.

Anschließend wird das Wasser oder die wässrige Lösung zwecks Trocknung in an sich bekannter Weise entfernt. Dazu kann man die Matrix beispielsweise auf Saugpapier legen.

Einer bevorzugten Ausführungsform zufolge setzt man dem Gemisch aus Polymerpartikeln und Kochsalzpartikeln eine Polymerlösung zu und entfernt das Lösungsmittel, bevor man kompaktiert. Dabei können Polymerpartikel und Polymerlösung auf dem gleichen Polymer basieren. Es kann sich aber auch um unterschiedliche Polymere, insbesondere mit unterschiedlicher biologischer Abbaubarkeit handeln. Der von Polymerlösung hat den Vorteil, dass quasi Stützpfeiler in die Matrix eingezogen werden, wodurch sich die mechanischen Eigenschaften der Matrix verbessern lassen. Eine solche Matrix weist insbesondere eine geringere Neigung auf zu zerkrümeln.

Das verwendete Lösungmittel sollte das Polymer, nicht aber das Salz lösen. Dadurch ist gewährleistet, dass die porogenen Eigenschaften des Salzes nicht oder nur unwesentlich beeinflusst werden. Aceton, Ethylacetat, Methylenchlorid, Chloroform, Hexafluorisopropanol, chlorierte und fluorierte, aliphatische und aromatische Kohlenwasserstoffe, Tetrahydrofuran, Ethylmethylketon, Diethylketon sowie Gemische davon eignen sind beispielsweise zum Lösen der vorstehend genannten Polymere. Zum Lösen von Poly(glykolsäure), Poly(milchsäure) oder Poly(glykolsäuremilchsäure) und mit Blick auf die medizinische Verwendung eignet sich insbesondere Chloroform.

Gibt man die Polymerlösung und das Polymerpartikel/Salzpartikel-Gemisch zusammen, entsteht zunächst eine rührbarer Brei, der dann unter Entfernung des Lösungsmittel rasch fest wird. Konzentration des Polymers in der Lösung sind zweckmäßigerweise so zu wählen, dass einerseits das Polymer vollständig gelöst ist, andererseits das Lösungsmittel rasch entfernt werden kann ohne die Polymerpartikel in nennenswertem Umfang anzulösen.

Als günstig hat sich ein Gewichtsverhältnis von Polymerpartikeln zu gelöstem Polymer 10:1 bis 1:100, vorteilhafterweise 2:1 bis 1:25 und insbesondere 1:1 bis 1:10 erwiesen.

Was das Gewichtsverhältnis von Polymerpartikeln zu Kochsalzpartikeln angeht, so kann im Rahmen dieser Ausführungsform ein zugunsten von Kochsalz höheres Gewichtsverhältnis von bis zu 1:200, 1:500 oder 1:1000 gewählt werden, wobei das Gewichtsverhältnis von Gesamtpolymer zu Kochsalz nach wie größer als 1:100 ist. Auf diese Art und Weise gelingt es, Porositäten oberhalb von 98 % einzustellen.

Bei dem vorstehend beschriebenen Verfahren dient das Kochsalz als porogenes Material, womit definitionsgemäß ein festes oder zumindest halbfestes Material gemeint ist, das mit dem matrixbildenden Polymer zunächst zu einem Gemisch vereinigt und dann aus dem Gemisch wieder entfernt wird, wodurch Hohlräume (Poren) entstehen. Dazu ist es zweckmäßig, dass das porogene Material in wenigstens einem Lösungsmittel löslich und in wenigstens einem weiteren Lösungsmittel im Wesentlichen unlöslich ist. Im Wesentlichen unlöslich ist ein Material insbesondere dann, wenn es unter den Verarbeitungsbedingungen, d.h. in der Regel bei Temperaturen im Bereich von 18 °C bis 25 °C und unter Normaldruck, zu weniger als 30 Gew.-%, vorzugsweise zu weniger als 20 Gew.-%, insbesondere zu weniger als 10 Gew.-%, beispielsweise zu weniger als 5, 4, 3, 2 und 1 Gew.-% löslich ist.

Struktur und Eigenschaften der resultierenden Matrices werden wesentlich durch das zu ihrer Herstellung verwendete porogene Material bestimmt. Dabei spielen nicht nur die Art des porogenen Materials, sondern vor allem die Korngrößenverteilung der porogenen Partikel eine Rolle. So gilt im Allgemeinen, dass mit zunehmender Korngröße nicht nur die Porengröße, sondern auch die Konnektivität, d.h. das Netzwerk miteinander kommunizierender Hohlräume, zunimmt. Dieses Netzwerk, auch Makrostruktur oder makroporöse Struktur genannt, ist zu unterscheiden von den durch Schäumen erhältlichen Poren, die in der Regel geschlossen sind und daher eine als Mikrostruktur oder mikroporös bezeichnete Struktur ausbilden.

Verfahren zur Herstellung einer porösen Matrix auf Basis eines biologisch verträglichen Polymers oder Polymergemisches, dadurch gekennzeichnet, dass man ein Gemisch aus Polymerpartikeln, Partikeln eines porogenen Materials und einer Polymerlösung kompaktiert und anschließend das porogene Material herauslöst.

Diese Verfahren ist grundsätzlich nicht auf die zuvor beschriebenen Merkmale beschränkt. So kann das Polymer ausgewählt sein unter Polyanhydriden, Poly(orthoestern), Poly(α-hydroxyestern), Poly(esteramiden), Polyamiden, Poly(esterethern), Polycarbonaten, Polyalkylenen, Polyalkylenglykolen, Polyalkylenoxiden, Polyalkylenterephthalaten, Polyvinylalkoholen, Polyvinylethern, Polyvinylestern, Polyvinylhalogeniden, Polyvinylpyrrolidonen, Polysiloxanen, Polystyrolen, Polyurethanen, derivatisierten Cellulosen, (Meth)acrylsäurepolymeren und -copolymeren. Das porogene Material ist Natriumchlorid. Polymer, porogenes Material und das zur Bildung der Lösung verwendete Lösungsmittel sind grundsätzlich so aufeinander abzustimmen, dass die Lösung Polymer in gelöster und Polymerpartikel in fester Form enthält sowie das porogene Material im wesentlichen nicht löst.

Die mit den vorstehend beschriebenen Verfahren erhältlichen Matrices sind ebenfalls Gegenstand der vorliegenden Erfindung.

Ein weiterer Gegenstand der vorliegenden Erfindung sind Implantate, die wenigstens eine der vorstehend beschriebenen Matrices und wenigstens eine Zelle umfassen. Je nach Zweck des Implantats können die Zellen dabei insbesondere ausgewählt sein unter Leberzellen, Pankreaszellen, Fettzellen, Darmzellen, Hautzellen, Gefäßzellen, Nervenzellen, Muskelzellen, Schilddrüsenzellen und Zahnwurzelzellen. Besondere Ausführungsformen erfindungsgemäßer Implantate betreffen Leberzellen und Pankreaszellen.

Ein weiterer Gegenstand der vorliegenden Erfindung sind Implantate, die wenigstens eine Matrix auf Basis eines biologisch verträglichen Polymers und Zellen wenigstens zweier Zelltypen umfassen, wobei die Zellen des ersten Zelltyps Hepatozyten und die Zellen des zweiten Zelltyps Langerhans'sche Inselzellen sind. Dieser Gegenstand ist nicht auf die vorstehend beschriebenen Matrices, d.h. Implantate auf Basis der erfindungsgemäßen Matrices, beschränkt.

Je nach Zweck des Implantats, d.h. insbesondere der zu erfüllenden Funktion, sind bestimmte Verhältnisse von Hepatozyten zu Langerhans'schen Inselzellen von Vorteil. So betrifft eine Ausführungsform der Erfindung Implantate, die nach Implantation die endokrinen Eigenschaften eines äquivalenten Pankreasorgans zeigen. Hierfür hat sich ein Verhältnis von Hepatozyten zu Langerhans'schen Inselzellen von etwa 10⁶: 3000 als vorteilhaft erwiesen. Eine weitere Ausführungsform der Erfindung betrifft Implantate, die nach Implantation Stoffwechselfunktionen einer Leber vollziehen. Hierfür hat sich ein Verhältnis von Hepatozyten zu Langerhans'schen Inselzellen von etwa 10⁶: 3-200, vorteilhafterweise von 10⁶: 10-100, insbesondere von 10⁶: 20-80 und besonders bevorzugt von etwa 10⁶ : 35-45 als zweckmäßig erwiesen.

Es sei angemerkt, dass derartige Implantate in der Regel neben Hepatozyten und Langerhans'schen Inselzellen weitere Zellen beinhalten, nämlich insbesondere weitere Leber- und Pankreaszellen, die bei der Zellisolation mit anfallen.

Die zur Besiedlung erfindungsgemäßer Matrices zu verwendenden Zellen oder Zellgemische können in an sich bekannter Weise gewonnen werden. Im Sinne eines autologen Implantats stammen die Zellen vorzugsweise aus dem Individuum, dem das Implantat eingesetzt werden soll. So wird dem Individuum in der Regel geeignetes Gewebe, beispielsweise ein Stück Leber oder Pankreas, entnommen und in geeigneter Weise für die Beimpfung und *in vitro*-Kultur der Matrix vorbereitet. Hierbei ist es von Bedeutung, dass die Zellen eine möglichst hohe Vitalitätsrate aufweisen.

Gewinnt man Leberzellen aus Lebergewebe, ist zu beachten, dass die Leberzellen insbesondere im Falle einer Leberzirrhose von einer starken Bindegewebsschicht umgeben sind. Um die Leberzellen mit einem möglichst hohen Anteil vitaler Zellen isolieren zu können, werden erfindungsgemäß Lösungen bestimmter Zusammensetzung verwendet.

Eine NaCl, KCl und HEPES enthaltende, wässrige Zusammensetzung A mit einem pH-Wert von etwa 7,4 ist nützlich für die Verwendung zum Perfundieren eines Leber-oder Pankreasstücks. Insbesondere enthalten 1000 ml dieser Lösung etwa 8,3 g NaCl, 0,5 g KCl und 2,38 g HEPES. Das Perfundieren erfolgt vorzugsweise bei einer Temperatur von etwa 37 °C und einer Flussrate von etwa 30 ml/min. Wenige Minuten, insbesondere etwa 5 bis 120 Minuten, beispielsweise etwa 7 Minuten, reichen aus, um bei der vorstehend genannten Flussrate das Gewebestück ausreichend zu perfundieren.

Alternativ dazu kann man auch eine Ethylenglykoltetraessigsäure (EGTA) enthaltende, wässrige Zusammensetzung A' zum Perfundieren eines Leber- oder Pankreasstücks verwenden.

Eine wässrige Zusammensetzung B mit einem pH-Wert von etwa 7,3 bis 7,4, vorzugsweise etwa 7,35, welche NaCl, KCl, HEPES, CaCl₂, Collagenase und Trypsininhibitor enthält, ist nützlich für die Verwendung zum Perfundieren eines Leber- oder Pankreasstücks. Vorzugweise enthalten 1000 ml der Lösung 8,3 g NaCl, 0,5 g KCl, 2,38 g HEPES, 0,7 g CaCl₂ x 2 H₂O, 500 mg Collagenase H und 7,5 mg Trypsininhibitor. Auch in diesem Fall hat sich das Perfundieren bei etwa 37 °C und einer Flussrate von etwa 30 ml/min als zweckmäßig erwiesen. Wenige Minuten, insbesondere etwa 5 bis 10 Minuten, beispielsweise etwa 6 bis 7 Minuten, reichen aus, um das Gewebestück hinreichend zu perfundieren.

Alternativ dazu kann man auch eine wässrige Zusammensetzung B' zum Perfundieren eines Leber- oder Pankreasstücks verwenden, welche Kollagenase und Hyaluronidase enthält. Vorzugsweise enthalten 1000 ml der Lösung 5 bis 10 U/ml Kollagenase und 5 bis 10 U/ml Hyaluronidase.

Es ist für die Vitalität der zu gewinnenden Zellen von Vorteil, wenn das Gewebestück zunächst mit Zusammensetzung A und anschließend mit Zusammensetzung B behandelt wird. Alternativ dazu kann zunächst eine Zusammensetzung A' und dann eine Zusammensetzung B' verwendet werden.

Im Anschluss an die Perfusion kann das Gewebestück dann frei präpariert werden und in einem geeigneten Medium, beispielsweise Williams-Medium E, vorsichtig aufgeschüttelt werden. Enthält die resultierende Zellsuspension noch gröbere Zelltrümmer, können diese in an sich bekannter Weise entfernt werden, beispielsweise indem man die Zellsuspension über ein Nylonnetz (200 µm) filtriert. Die Zellen des Filtrats können dann vorsichtig pelletiert werden, wobei sich eine dreiminütige Zentrifugation bei 50 g und 4 °C als vorteilhaft erwiesen hat.

Die Auftragung der gewonnenen Zellen auf die Matrices erfolgt in an sich bekannter Weise. In der Regel werden die Zellen als zellhaltige Lösung auf die Matrix aufgetragen und anschließend - üblicherweise unter Zellkulturbedingungen - inkubiert, bis Zellen an der Matrix anhaften. Werden mehr als ein Zelltyp, beispielsweise Hepatocyten und Langerhans'sche Inselzellen, auf eine Matrix aufgetragen, können die verschiedenen Zelltypen im Prinzip gemeinsam oder aber nacheinander aufgetragen werden. Einer besonderen Ausführungesform zufolge trägt man zunächst Langerhans'schen Inselzellen und anschließend Hepatocyten auf, wobei man nach dem Auftragen jeweils inkubiert, bis zumindest ein Teil der Zellen an der Matrix anhaftet.

Erfindungsgemäße Matrices und Implantate weisen entscheidende Vorteile auf. So ermöglichen die inneren Dimensionen der Matrices eine effiziente Besiedlung mit Zellen. Die Matrices sind einerseits frei verformbar und bieten andererseits ausreichend Stabilität und Rigidität, um die chirurgische Implantationsprozedur zu überstehen und den am Implantationsort einwirkenden mechanischen Kräften zu widerstehen. Der nach der Implantation einsetzende, initiale Zelluntergang ist begrenzt und implantiertes Gewebe kann nach kurzer Zeit die beabsichtigte Funktion aufnehmen. Kurz nach der Implantation kommt es zur Gefäßeinsprossung bzw. zur Einsprossung von gefäßreichem Granulationsgewebe und auch von Nervengewebe. Die erfindungsgemäßen Matrices können hergestellt werden, ohne physiologisch bedenkliche Lösungsmittel, beispielsweise Formaldehyd, verwenden zu müssen, so dass kein spezielles Verfahren zur Elimination der Lösungsmittel erforderlich ist und die Gefahr verbleibender Restmengen dieser Lösungsmittel nicht besteht.

Erfindungsgemäße Matrices und Implantate weisen vielfältige Verwendungsmöglichkeiten auf. Hiervon sind insbesondere Verwendungen im medizinischen Bereich zu nennen. Ein weiterer Gegenstand der vorliegenden Erfindung sind daher die erfindungsgemäßen Matrices und Implantate zur therapeutischen Verwendung.

Eine besondere Verwendung in diesem Bereich ist die des Aufbaus von Gewebe (Tissue Engineering). Dabei dienen die erfindungsgemäßen Matrices quasi als Gerüst (Scaffold), in das Zellen einwandern und/oder sich anheften.

Dazu kann man die Matrices beispielsweise in vitro mit den gewünschten Zellen beimpfen, z.B. mit einer zellhaltigen Lösung versetzen und inkubieren, bis Zellen sich an die Matrix angeheftet haben. Eine solche Matrix mit daran anhaftenden Zellen (hier mit Implantat bezeichnet) kann dann weiteren Verfahrensmaßnahmen, beispielsweise weiterer Kultivierung, gegebenenfalls unter Einwirkung von Wirkstoffen, z. B. zur weiteren Expansion der Zellen oder zur Modulierung ihrer Eigenschaften, unterzogen werden, und/oder bis zur Implantation in geeigneter Weise, beispielsweise auf Eis oder in einem Bioflussreaktor unter Standardbedingungen, aufbewahrt werden. Im Rahmen dieser Verwendung ist es von Vorteil, die zur Implantation bestimmten Zellen zunächst in vitro isolieren und gegebenenfalls auch expandieren zu können. Insbesondere wird dadurch das Aufbringen verschiedener Zelltypen, wie die oben beschriebenen Hepatozyten zusammen mit Langerhans'schen Inselzellen, auf eine Matrix ermöglicht.

Anstelle einer in vitro Beimpfung besteht eine weitere Möglichkeit darin, die Matrix (ohne vorherige Zellanheftung) zu implantieren mit dem Ziel, zur Geweberegenerierung befähigte Vorläuferzellen zu veranlassen, in ein geschädigtes Gewebe einzuwandern und dort verloren gegangenes Gewebe zu regenerieren. Die Matrix muss dazu so gestaltet sein, dass erwünschte Zellen, nicht aber unerwünschte Zellen, in die Matrix einwandern können. Eine derartige Verwendung wird im Allgemeinen als geführte Geweberegenerierung (GTR für "Guided Tissue Regeneration") bezeichnet.

Eine erfindungsgemäße Matrix oder ein erfindungsgemäßes Implantat kann daher zur Verwendung bei der Behandlung des menschlichen oder tierischen Körpers dienen. Dazu werden eine oder mehrere Matrices bzw. ein oder mehrere Implantate dem zu behandelnden Körper im Wege eines chirurgischen Eingriffs eingesetzt. Beinhaltet das Implantat Zellen mit Organfunktion bzw. sollen in die Matrix Zellen mit Organfunktion einwandern, wir es beispielsweise bei Hepatozyten oder Langerhans'schen Inselzellen der Fall ist, können die Matrices oder Implantate beispielsweise in das Mesenterium, subkutane Gewebe, Retroperitoneum, den properitonealen Raum oder den intramuskulären Raum des zu behandelnden Individuums implantiert werden.

Mit den erfindungsgemäßen Matrices bzw. Implantaten können im Prinzip sämtliche Individuen behandelt werden, die einen entsprechenden Gewebeersatz benötigen. Dies sind in der Regel Individuen, die an einer bestimmten Störung oder Erkrankung leiden, in deren Verlauf es zum Verlust von funktionsfähigem Gewebe kommt. Dies kann unter Umständen ganze Organe, beispielsweise die Leber oder das Pankreas betreffen. So richtet sich die vorliegende Erfindung insbesondere auf die Verwendung bei der Behandlung von Erkrankungen, die zu chronischem Leber- oder Pankreasversagen führen. Hierzu gehören beispielsweise chronische Hepatitis und biliäre Zirrhose bei Erwachsenen sowie biliäre Atresie und angeborene metabolische Defekte bei Kindern. Auch bei Leberkarzinomen kann eine Lebertransplantation angezeigt sein. Eine Pankreastransplantation hingegen ist insbesondere angezeigt bei allen Formen eines Diabetes mellitus, insbesondere einem vom Typ I oder II.

Eine erfindungsgemäße Matrix oder ein erfindungsgemäßes Implantat ist nützlich bei der Bereitstellung eines therapeutischen Mittels zur Transplantation eines Individuums und dabei insbesondere zur Behandlung eines Individuums, das an einem zumindest partiellen Verlust funktionsfähigen Gewebes leidet, welches durch das Transplantat ersetzt werden soll.

Die folgenden Beispiele sollen die Erfindung veranschaulichen, ohne sie in ihrem Umfang einzuschränken.

### Beispiel 1

### Herstellung der Matrix

### a) Ohne Polymerlösung

Polymer-Pellets (Resomer ® RG 858, erhältlich von der Firma Boehringer,
Ingelheim) werden in Flüssigstickstoff gefroren und in gefrorenem Zustand geschreddert (Schlagwerk der Firma Däschle; 12000 U/min 2 min). Die geschredderten Polymerpartikel werden gesiebt. Partikel mit einer Größe von 108 µm bis 250 µm werden für die Matrixherstellung eingesetzt. Dabei besitzen 16 Gew.-% des eingesetzten Polymers eine Partikelgröße zwischen 108 µm und 140 µm, 22 Gew.-% des eingesetzten Polymers eine Partikelgröße zwischen 145 µm und 180 µm, 46 Gew.-% des eingesetzten Polymers eine Partikelgröße zwischen 185 µm und 220 µm, und 16 Gew.-% des eingesetzten Polymers eine Partikelgröße zwischen 225 µm und 250 µm. Kochsalz wird gesiebt und Kochsalzpartikel mit einer Korngröße von 250 µm bis 425 µm werden für die Matrixherstellung verwendet. Dabei besitzen 25 Gew.-% des eingesetzten Salzes eine Partikelgröße zwischen 250 µm und 320 µm, 44 Gew.-% des eingesetzten Salzes eine Partikelgröße zwischen 330 µm und 380 µm, und 31 Gew.-% des eingesetzten Salzes eine Partikelgröße zwischen 390 µm und 425 µm. 760 mg Kochsalzpartikel und 40 mg Polymerpartikel werden miteinander vermischt. Das Gemisch wird in eine Stanzform eingebracht und mit einer Hydraulikpresse bei einem Stempeldruck von 1000 psi 1 Minute gepresst. Anschließend werden die Matrixrohlinge auf einen Teflonteller gelegt und 24 Stunden in einer CO-Atmosphäre (850 psi) begast. Dann werden die Rohlinge 24 Stunden gewässert, um die eingeschlossenen Salzkörner aufzulösen. Schließlich werden die Matrices 12 Stunden auf Saugpapier getrocknet.

Die resultierende Polymermatrix weist eine Porosität von 95 +/- 2% und eine mittels Rasterelektronenmikroskopie bestimmte, definierte Porengröße von 250 µm +/- 120 µm auf.

### b) Mit Polymerlösung

Kochsalz (analytisch rein) wird gemahlen (Schlagwerk der Firma Däschle; 12000 Ulmin 2 min) und anschließend gesiebt, und Kochsalzpartikel mit einer Korngröße von 108 bis 425 µm werden für die Matrixherstellung verwendet. Dabei besitzen 8 % des eingesetzten Salzes eine Partikelgröße zwischen 108 µm und 140 µm, 6 Gew.-% des eingesetzten Salzes eine Partikelgröße zwischen 145 µm und 180 µm, 12 Gew.-% des eingesetzten Salzes eine Partikelgröße zwischen 185 µm und 220 µm, 6 Gew.-% des eingesetzten Salzes eine Partikelgröße zwischen 225 µm und 250 µm, 25 Gew.-% des eingesetzten Salzes eine Partikelgröße zwischen 250 µm und 320 µm, 26 Gew.-% des eingesetzten Salzes eine Partikelgröße zwischen 330 µm und 380 µm, und 17 Gew.-% des eingesetzten Salzes eine Partikelgröße zwischen 390 µm und 425 µm. 96 g Kochsalzpartikel werden mit 1 g der in Beispiel 1 a) beschriebenen Polymerpartikel vermischt und anschließend mit 100 ml einer Chloroformlösung, die 4 g des Polymers gelöst enthält, versetzt. Das so erhaltene Gemisch wird bei 45 °C bis 65 °C erwärmt, wodurch das Chloroform innerhalb von etwa 25 Minuten verdampft. Das verbleibende Salz-Polymer-Gemisch wird dann mit einer Hydraulikpresse bei einem Stempeldruck von 1000 psi eine Minute gepresst und anschließend 24 Stunden gewässert, um die eingeschlossenen Salzkörner aufzulösen. Anschließend wird die Matrix, wie oben beschrieben, begast und schließlich 12 Stunden auf Saugpapier getrocknet.

Die resultierende Polymermatrix weist eine Porosität von 96 % auf.

Vermischt man 98,5 g Salzpartikel mit 0,5 g Polymerpartikel und versetzt das Gemisch mit 100 ml einer Chloroform-Lösung, die 1 g Polymer enthält, erhält man eine Matrix mit einer Porosität von 99 %.

Vermischt man 99,2 g Salzpartikel mit 0,1 g Polymerpartikel und versetzt dieses Gemisch mit 100 ml einer Chloroform-Lösung, die etwa 0,9 g Polymer enthält, erhält man eine Polymermatrix mit einer Porosität von 99 %.

### Beispiel 2

### a) Beschichtung der Matrix mit Fibronectin

Die Matrix aus Beispiel 1 wird in eine 3 µg/ml Fibronectin aus Humanplasma (Sigma) enthaltende Carbonatpufferlösung mit einem pH-Wert von 9,4 getaucht. Nach etwa 60 s wird die Matrix aus der Lösung entnommen, lyophilisiert und γ-sterilisiert.

### Beispiel 3

### Zellisolation

Dem zu transplantierenden Individuum wird in an sich bekannter Weise ein Leberstück entnommen. Das entnommene Leberstück wird zunächst 7 Minuten bei einer Flussrate von 30 ml/min und 37 °C mit einer Lösung (8,3 g NaCl; 0,5 g KCl; 2,38 g HEPES; ad 1000 ml destilliertes Wasser; pH-Wert 7,4) perfundiert. Anschließend wird das Leberstück weitere 6 bis 7 min bei einer Flussrate von 30 ml/min und 37 °C mit einer Collagenase-Trypsin-Inhibitor-Lösung (8,3 g NaCl; 0,5 g KCl; 2,38 g HEPES; 0,7 g CaCl₂ x 2 H₂O; 500 mg Collagenase (Collagenase H, Boehringer Mannheim, Mannheim, Deutschland); 7,5 mg Trypsin-Inhibitor (ICN, Eschwege, Deutschland); ad 1000 ml destilliertes Wasser; pH-Wert 7,35) perfundiert. Nach Beendigung der Perfusion wurde das Leberstück frei präpariert und in Williams-Medium E vorsichtig aufgeschüttelt. Die Zellsuspension wird filtriert (Nylonnetz; 200 µm) und anschließend mit Williams-Medium E gewaschen. Anschließend werden die Zellen bei 3 min bei 50 g und 4 °C zentrifugiert. Die mit Tryptan-Blau bestimmte Vitalität der Zellen beträgt 95 %.

In gleicher Weise werden Langerhans'sche Inselzellen aus einem Pankreasstück isoliert.

### Beispiel 4

### Zellbesiedlung

Die in Beispiel 2 beschichteten Matrices werden im ersten Schritt mit Langerhans'schen Inselzellen inkubiert, welche gemäß Beispiel 3 isoliert wurden.

Dazu wurden 3000 Inselzellen pro ml in einem Lösungsgemisch aus M199 und FKS (Volumenverhältnis von 19:1) suspendiert. Die Zellzahl wird bestimmt, indem man sie unter einem inversen Olympus-Mikroskop in einem 0,25-mm-Zählrohr ausgezählt. Dann werden 8 ml bis 10 ml dieser Lösung mit einer Pipette auf die Matrix aufgebracht. Die überschüssige Lösung, welche nicht in der Matrix verbleibt, wird verworfen. Die so behandelte Matrix wird anschließend zur Anheftung der Zellen 4 Stunden in den Zellkulturbrutschrank gestellt. Anschließend wird eine Lösung aus Williams Medium E, die pro ml eine nicht gereinigte Leberzellsuspension mit etwa 5,0 x 10⁷ vitalen Hepatocyten und etwa 1,0 x 10⁶ nichtperenchymatösen Leberzellen enthält, auf die Matrix aufgebracht. Es werden 8 ml bis 12 ml Lösung mit einer Pipette aufgetragen; die nicht von der Matrix aufgenommene überschüssige Lösung wird verworfen. Die Matrix kann bis zur Implantation etwa 1,5 Stunden auf Eis gehalten werden. Ist eine Implantation zu einem späteren Zeitpunkt vorgesehen, kann die Matrix in einem Bioflussreaktor unter Standardbedingungen bis zu 5 Tagen aufbewahrt werden.

### Beispiel 5

### Sekretorische Aktivität und Proliferationsrate der Hepatozyten

Lewis-Ratten wurden mit zellbesiedelten Matrices nach Beispiel 4 transplantiert. Die Transplantate wurden den Tieren zu verschiedenen Zeitpunkten wieder entnommen und morphometrisch untersucht. Die Zellzahl der die Form einer kreisrunden Scheibe mit einem Durchmesser von 15 mm und einer Dicke von 2 mm aufweisenden Transplantate betrug 1, 6 und 12 Monate nach Transplantation 94 x 10³, 140 x 10³ bzw. 146 x 10³ Zellen. Hepatozyten des einen Monat nach Transplantation entnommenen Transplantats weisen eine normale Albumin-Expression auf. In allen Präparaten werden proliferierende Hepatozyten gefunden, ohne dass eine pathologisch erhöhte Proliferationsrate vorliegt. Im Vergleich zu Leberstandardpräparaten weisen die erfindungsgemäß transplantierten Hepatozyten einen um den Faktor 3 erhöhten Einbau von BrdU auf.

### Beispiel 6

### Vaskularisierung

Die weitere Untersuchung der in Beispiel 4 beschriebenen Matrices ergibt, dass diese bereits einen Monat nach Implantation hervorragend vaskularisiert sind. Die Blutgefäße reichen makroskopisch bis zur Matrix und die transplantierten Hepatozyten und Langerhans'sche Inselzellen bekommen durch eine ausreichende Kapillarisierung Kontakt zum kardiovaskulären System des Transplantatempfängers.

Es ist weiterhin festzustellen, dass die cotransplantierten Langerhans'sche Inselzellen beim Empfänger keine Hypoglykämie verursachen. Die endokrine Sekretionsleistung dieser Zellen sowie der empfängereigenen Inselzellen wird vermutlich durch einen Rückkopplungsmechanismus geregelt.

### Beispiel 7

### Leberfunktionsübernahme

Gunn-Ratten gelten als Tiermodell für das menschliche Crigler-Najar-Syndrom, da ihre Leber infolge eines spezifischen angeborenen metabolischen Enzymdefekts nicht ausreichend Bilirubin konjugieren kann. Als Folge führen toxische Blutplasmaspiegel unkonjugierten Bilirubins über zahlreiche Folgeschäden zum Tod.

Drei Gunn-Ratten werden mit einer zellbesiedelten Matrix gemäß Beispiel 4 transplantiert. Die Matrix besitzt eine Außenfläche von insgesamt 10 cm².

Bereits vier Wochen nach Transplantation sinkt der Bilirubinspiegel der Versuchstiere. Bilirubin wird nunmehr konjugiert. Das konjugierte Bilirubin kann in allen drei Fällen in den Gallengängen der noch vorhandenen Leber mit Hilfe einer Gallengangsonde nachgewiesen werden. Somit gelangt das in der Matrix konjugierte Bilirubin hämatogen in die Leber und kann dort über das Gallengangsystem ausgeschieden werden.

### Beispiel 8

### Humane Patienten

Ein Patient mit einer ausgeprägten Leberzirrhose wird mit zellbesiedelten Matrices gemäß Beispiel 4 in die Bauchhöhle transplantiert. Die folgende Tabelle 1 fasst die Laborbefunde des Patienten vor der Transplantation zusammen.

**Tabelle 1**

| Parameter | Patient 1 |
|---|---|
| GOT | 27 |
| GPT | 35 |
| gGt | 89 |
| CHE | 2421 |
| Serumalbumin | 24,1 |

Patient (Ethyltoxische Leberzirrhose, zuvor mehrfach dekompensiert, jetzt nicht aktiv) erhielt 4 Matrices (je 124 mm x 45 mm x 5 mm).

Die folgende Tabelle 2 fasst die Leberwerte 3, 10 bzw. 20 Wochen nach Transplantation zusammen.

**Tabelle 2**

| | Patient 1 | | |
|---|---|---|---|
| | 3 | 10 | 20 |
| GOT | 22 | 10 | 11 |
| GPT | 28 | 9 | 28 |
| gGt | 71 | 10 | 9 |
| Serumalbumin | 28,6 | 42 | 44 |
| CHE | 2652 | 4400 | 4600 |

## Patentansprüche

1. Poröse Matrix auf Basis eines biologisch verträglichen Polymers oder Polymergemisches, **dadurch gekennzeichnet, dass** die Matrix Poren mit einer Größe von 130 µm oder weniger und Poren mit einer Größe von 370 µm oder mehr aufweist, der Porositätsgrad 93 bis 98 % beträgt und die Matrix durch Kompaktieren eines Gemisches aus Polymerpartikeln mit einer Korngröße im Bereich von 20 bis 950 µm und Kochsalzpartikeln mit einer Korngröße im Bereich von 90 bis 670 µm und anschließendes Herauslösen des Kochsalzes erhältlich ist, wobei der Porositätsgrad die zahlenmäßige Angabe in % zum Anteil des Porenvolumens am Gesamtvolumen der Matrix ist und wobei
die Matrix folgende Porengrößenverteilung aufweist: 0,5 % bis 6 % Poren mit einem mittleren Durchmesser im Bereich von 70 bis 100 µm; 2 % bis 8 % Poren mit einem mittleren Durchmesser im Bereich von 101 bis 115 µm; 2 % bis 8 % Poren mit einem mittleren Durchmesser im Bereich von 116 bis 130 µm; 1 % bis 7 % Poren mit einem mittleren Durchmesser im Bereich von 131 bis 300 µm; 11 % bis 23 % Poren mit einem mittleren Durchmesser im Bereich von 301 bis 330 µm; 4 % bis 10 % Poren mit einem mittleren Durchmesser im Bereich von 331 bis 360 µm; 5 % bis 17 % Poren mit einem mittleren Durchmesser im Bereich von 361 bis 390 µm; 7 % bis 19 % Poren mit einem mittleren Durchmesser im Bereich von 391 bis 420 µm; 3 % bis 9 % Poren mit einem mittleren Durchmesser im Bereich von 421 bis 450 µm, 12 % bis 24 % Poren mit einem mittleren Durchmesser im Bereich von 451 bis 480 µm; und 5 % bis 17 % Poren mit einem mittleren Durchmesser im Bereich von 481 bis 510 µm aufweist,
wobei die Porengrößen und die Porengrößenverteilung durch Rasterelektronenmikroskopie bestimmt werden.

2. Matrix nach Anspruch 1, **dadurch gekennzeichnet, dass** die Matrix folgende Porengrößenverteilung aufweist: 1 % bis 5 % Poren mit einem mittleren Durchmesser im Bereich von 70 bis 100 µm; 3 % bis 7 % Poren mit einem mittleren Durchmesser im Bereich von 101 bis 115 µm; 3 % bis 7 % Poren mit einem mittleren Durchmesser im Bereich von 116 bis 130 µm; 2 % bis 6 % Poren mit einem mittleren Durchmesser im Bereich von 131 bis 300 µm; 13 % bis 21 % Poren mit einem mittleren Durchmesser im Bereich von 301 bis 330 µm; 5 % bis 9 % Poren mit einem mittleren Durchmesser im Bereich von 331 bis 360 µm; 7 % bis 15 % Poren mit einem mittleren Durchmesser im Bereich von 361 bis 390 µm; 9 % bis 17 % Poren mit einem mittleren Durchmesser im Bereich von 391 bis 420 µm; 4 % bis 8 % Poren mit einem mittleren Durchmesser im Bereich von 421 bis 450 µm; 14 % bis 22 % Poren mit einem mittleren Durchmesser im Bereich von 451 bis 480 µm; und 7 % bis 15 % Poren mit einem mittleren Durchmesser im Bereich von 481 bis 510 µm aufweist.

3. Matrix nach Anspruch 1, **dadurch gekennzeichnet, dass** die Matrix folgende Porengrößenverteilung aufweist: 2 % bis 4 % Poren mit einem mittleren Durchmesser im Bereich von 70 bis 100 µm; 4 % bis 6 % Poren mit einem mittleren Durchmesser im Bereich von 101 bis 115 µm; 4 % bis 6 % Poren mit einem mittleren Durchmesser im Bereich von 116 bis 130 µm; 3 % bis 5 % Poren mit einem mittleren Durchmesser im Bereich von 131 bis 300 µm; 15 % bis 19 % Poren mit einem mittleren Durchmesser im Bereich von 301 bis 330 µm; 6 % bis 8 % Poren mit einem mittleren Durchmesser im Bereich von 331 bis 360 µm; 9 % bis 13 % Poren mit einem mittleren Durchmesser im Bereich von 361 bis 390 µm; 11 % bis 15 % Poren mit einem mittleren Durchmesser im Bereich von 391 bis 420 µm; 5 % bis 7 % Poren mit einem mittleren Durchmesser im Bereich von 421 bis 450 µm; 16 % bis 20 % Poren mit einem mittleren Durchmesser im Bereich von 451 bis 480 µm; und 9 % bis 13 % Poren mit einem mittleren Durchmesser im Bereich von 481 bis 510 µm aufweist.

4. Matrix nach Anspruch 1, **dadurch gekennzeichnet, dass** die Matrix folgende Porengrößenverteilung aufweist: 3 % Poren mit einem mittleren Durchmesser im Bereich von 70 bis 100 µm; 5 % Poren mit einem mittleren Durchmesser im Bereich von 101 bis 115 µm; 5 % Poren mit einem mittleren Durchmesser im Bereich von 116 bis 130 µm; 4 % Poren mit einem mittleren Durchmesser im Bereich von 131 bis 300 µm; 17 % Poren mit einem mittleren Durchmesser im Bereich von 301 bis 330 µm; 7 % Poren mit einem mittleren Durchmesser im Bereich von 331 bis 360 µm; 11 % Poren mit einem mittleren Durchmesser im Bereich von 361 bis 390 µm; 13 % Poren mit einem mittleren Durchmesser im Bereich von 391 bis 420 µm; 6 % Poren mit einem mittleren Durchmesser im Bereich von 421 bis 450 µm; 18 % Poren mit einem mittleren Durchmesser im Bereich von 451 bis 480 µm; und 11 % Poren mit einem mittleren Durchmesser im Bereich von 481 bis 510 µm aufweist.

5. Matrix nach einem der Ansprüche 1 bis 4, **dadurch gekennzeichnet, dass** das biologisch verträgliche Polymer ein biologisch abbaubares Polymer ist, das ausgewählt ist unter natürlichen Polymeren, wie Albumin, Fibrinogen, Collagen, Gelatine, Chitin, Chitosan, Agarose, Alginat und synthetischen Polymeren, wie Polyanhydriden, Poly(ε-caprolacton) und Poly(α-hydroxyestern).

6. Matrix nach Anspruch 5, **dadurch gekennzeichnet, dass** das biologisch abbaubare Polymer Poly(glycolsäure-milchsäure) mit einem Milchsäure-Anteil von 85 mol-% und einem Glycolsäure-Anteil von 15 mol-% ist.

7. Matrix nach einem der Ansprüche 1 bis 6, **dadurch gekennzeichnet, dass** die Oberfläche der Matrix mit wenigstens einem extrazellulären Matrixprotein beschichtet ist, das ausgewählt ist unter Collagenen, Laminin und Fibronectin.

8. Matrix nach Anspruch 7, **dadurch gekennzeichnet, dass** die Beschichtung Fibronectin enthält oder dass die Beschichtung ein Gemisch aus Collagen vom Typ I, Laminin und Collagen vom Typ IV enthält.

9. Verfahren zur Herstellung einer porösen Matrix auf Basis eines biologisch verträglichen Polymers oder Polymergemisches nach einem der Ansprüche 1 bis 8, **dadurch gekennzeichnet, dass** man ein Gemisch aus Polymerpartikeln und Kochsalzpartikeln kompaktiert und anschließend das Kochsalz herauslöst, wobei
die Kochsalzpartikel aus einem Gemisch aus 22 bis 28 Gew.-% Partikel mit einer Korngröße von 250 µm bis 320 µm, 42 bis 46 Gew.-% Partikel mit einer Korngröße von 330 bis 380 µm, und 29 bis 33 Gew.-% Partikel mit einer Korngröße von 390 µm bis 425 µm bestehen oder aus einem Gemisch aus 7 bis 9 Gew.-% Partikel mit einer Korngröße von 108 µm bis 140 µm, 5 bis 7 Gew.-% Partikel mit einer Korngröße von 145 µm bis 180 µm, 10 bis 14 Gew.-% Partikel mit einer Korngröße von 185 µm bis 220 µm, 5 bis 7 Gew.-% Partikel mit einer Korngröße von 225 µm bis 250 µm, 22 bis 28 Gew.-% Partikel mit einer Korngröße von 250 µm bis 320 µm, 22 bis 28 Gew.-% Partikel mit einer Korngröße von 330 µm bis 380 µm, und 15 bis 19 Gew.-% Partikel mit einer Korngröße von 390 µm bis 425 µm bestehen, und
die Polymerpartikel aus einem Gemisch aus 14 bis 18 Gew.-% Partikel mit einer Korngröße von 108 µm bis 140 µm, 20 bis 24 Gew.-% Partikel mit einer Korngröße von 145 µm bis 180 µm, 43 bis 49 Gew.-% Partikel mit einer Korngröße von 185 µm bis 220 µm, und 14 bis 18 Gew.-% Partikel mit einer Korngröße von 225 µm bis 250 µm bestehen.

10. Verfahren nach Anspruch 9, **dadurch gekennzeichnet, dass** das Gewichtsverhältnis von Polymerpartikeln zu Kochsalzpartikeln 1:100 bis 1:10, vorteilhafterweise 1:50 bis 1:15 und insbesondere 1:20 bis 1:18 beträgt.

11. Verfahren nach Anspruch 9 oder 10, **dadurch gekennzeichnet, dass** man vor dem Kompaktieren dem Gemisch aus Polymerpartikeln und Kochsalzpartikeln eine Polymerlösung zusetzt und das Lösungsmittel entfernt.

12. Verfahren nach Anspruch 11, **dadurch gekennzeichnet, dass** das Lösungsmittel das Polymer, nicht aber das Salz löst.

13. Verfahren nach Anspruch 12, **dadurch gekennzeichnet, dass** das Lösungsmittel ausgewählt ist unter Aceton, Ethylacetat, Methylenchlorid, Chloroform, Hexafluorisopropanol, chlorierten und fluorierten, aliphatischen und aromatischen Kohlenwasserstoffen, Tetrahydrofuran, Ethylmethylketon, Diethylketon sowie Gemischen davon.

14. Verfahren nach Anspruch 13, **dadurch gekennzeichnet, dass** das Polymer Poly(glykolsäure), Poly(milchsäure) oder Poly(glykolsäuremilchsäure) und das Lösungsmittel Chloroform ist.

15. Verfahren nach einem der Ansprüche 11 bis 14, **dadurch gekennzeichnet, dass** das Gewichtsverhältnis von Polymerpartikeln zu gelöstem Polymer 10:1 bis 1:100, vorteilhafterweise 2:1 bis 1:25 und insbesondere 1:1 bis 1:10 beträgt.

16. Verfahren nach Anspruch 9, **dadurch gekennzeichnet, dass** das Kompaktieren durch Einwirken von Druck erfolgt.

17. Verfahren nach Anspruch 9, **dadurch gekennzeichnet, dass** man zum Herauslösen des Kochsalzes Wasser auf das kompaktierte Gemisch einwirken lässt.

18. Verfahren nach Anspruch 17, **dadurch gekennzeichnet, dass** man das Wasser wieder entfernt.

19. Verfahren nach Anspruch 9, **dadurch gekennzeichnet, dass** das kompaktierte Gemisch zunächst in einer CO₂-Atmosphäre gelagert und anschließend das Kochsalz herauslöst wird.

20. Verfahren nach einem der Ansprüche 9 bis 19, **dadurch gekennzeichnet, dass** man ein Gemisch aus Polymerpartikeln, Kochsalzpartikeln und einer Polymerlösung kompaktiert und anschließend das Kochsalz herauslöst.

21. Verfahren nach Anspruch 20, **dadurch gekennzeichnet, dass** das Polymer ausgewählt ist unter Polyanhydriden, Poly(orthoestern), Poly(α-hydroxyestern), Poly(esteramiden), Polyamiden, Poly(esterethern), Polycarbonaten, Polyalkylenen, Polyalkylenglykolen, Polyalkylenoxiden, Polyalkylenterephthalaten, Polyvinylalkoholen, Polyvinylethern, Polyvinylestern, Polyvinylhalogeniden, Polyvinylpyrrolidonen, Polysiloxanen, Polystyrolen, Polyurethanen, derivatisierten Cellulosen, (Meth)acrylsäurepolymeren und -copolymeren.

22. Verfahren nach Anspruch 20, **dadurch gekennzeichnet, dass** die Lösung Polymer in gelöster und Polymerpartikel in fester Form enthält.

23. Verfahren nach Anspruch 20, **dadurch gekennzeichnet, dass** die Lösung das Kochsalz nicht löst.

24. Implantat, umfassend eine Matrix nach einem der Ansprüche 1 bis 8 und wenigstens eine Zelle.

25. Implantat nach Anspruch 24, umfassend eine Matrix auf Basis eines biologisch verträglichen Polymers, und Zellen wenigstens zweier Zelltypen, **dadurch gekennzeichnet, dass** die Zellen des ersten Zelltyps Hepatozyten und die Zellen des zweiten Zelltyps Langerhans'sche Inselzellen sind.

26. Implantat nach Anspruch 25, **dadurch gekennzeichnet, dass** das Verhältnis von Hepatozyten zu Langerhans'schen Inselzellen 10⁶ : 3000 beträgt.

27. Implantat nach Anspruch 25, **dadurch gekennzeichnet, dass** das Verhältnis von Hepatozyten zu Langerhans'schen Inselzellen 10⁶ : 3-200, vorteilhafterweise 10⁶ : 10-100, insbesondere: 20-80 und besonders bevorzugt 10⁶ : 35-45 beträgt.

## Claims

1. A porous matrix based on a biologically tolerated polymer or polymer mixture, **characterized in that** the matrix possesses pores having a size of 130 *µ*m or less and pores having a size of 370 *µ*m or more and the degree of porosity is from 93 to 98%, wherein the matrix is obtainable by compacting a mixture of polymer particles having a particle size in the range from 20 to 950 *µ*m and sodium chloride particles having a particle size in the range from 90 to 670 *µ*m and then dissolving out the sodium chloride, wherein the matrix has the following pore size distribution:
0.5% to 6% pores having a mean diameter in the range of from 70 to 1.00 *µ*m; 2% to 8% pores having a mean diameter in the range of from 101 to 115 *µ*m; 2% to 8% pores having a mean diameter in the range of from 116 to 130 *µ*m; 1% to 7% pores having a mean diameter in the range of from 131 to 300 *µ*m; 11% to 23% pores having a mean diameter in the range of from 301 to 330 *µ*m; 4% to 10% pores having a mean diameter in the range of from 331 to 360 *µ*m; 5% to 17% pores having a mean diameter in the range of from 361 to 390 *µ*m; 7% to 19% pores having a mean diameter in the range of from 391 to 420 *µ*m; 3% to 9% pores having a mean diameter in the range of from 421 to 450 *µ*m; 12% to 24% pores having a mean diameter in the range of from 451 to 480 *µ*m; and 5% to 17% pores having a mean diameter in the range of from 481 to 510 *µ*m,
wherein the pore size and the pore size distribution are determined by scanning electron microscopy.

2. The matrix as claimed in claim 1, **characterized in that** the matrix comprises 1% to 5% pores having a mean diameter in the range of from 70 to 100 *µ*m; 3% to 7% pores having a mean diameter in the range of from 101 to 115 *µ*m; 3% to 7% pores having a mean diameter in the range of from 116 to 130 *µ*m; 2% to 6% pores having a mean diameter in the range of from 131 to 300 *µ*m; 13% to 21% pores having a mean diameter in the range of from 301 to 330 *µ*m; 5% to 9% pores having a mean diameter in the range of from 331 to 360 *µ*m; 7% to 15% pores having a mean diameter in the range of from 361 to 390 *µ*m; 9% to 17% pores having a mean diameter in the range of from 391 to 420 *µ*m; 4% to 8% pores having a mean diameter in the range of from 421 to 450 *µ*m; 14% to 22% pores having a mean diameter in the range of from 451 to 480 *µ*m; and 7% to 15% pores having a mean diameter in the range of from 481 to 510 *µ*m.

3. The matrix as claimed in claim 1, **characterized in that** the matrix comprises 2% to 4% pores having a mean diameter in the range of from 70 to 100 *µ*m; 4% to 6% pores having a mean diameter in the range of from 101 to 115 *µ*m; 4% to 6% pores having a mean diameter in the range of from 116 to 130 *µ*m; 3% to 5% pores having a mean diameter in the range of from 131 to 300 *µ*m; 15% to 19% pores having a mean diameter in the range of from 301 to 330 *µ*m; 6% to 8% pores having a mean diameter in the range of from 331 to 360 *µ*m; 9% to 13% pores having a mean diameter in the range of from 361 to 390 *µ*m; 11% to 15% pores having a mean diameter in the range of from 391 to 420 *µ*m; 5% to 7% pores having a mean diameter in the range of from 421 to 450 *µ*m; 16% to 20% pores having a mean diameter in the range of from 451 to 480 *µ*m; and 9% to 13% pores having a mean diameter in the range of from 481 to 510 *µ*m.

4. The matrix as claimed in claim 1, **characterized in that** the matrix comprises 3% pores having a mean diameter in the range of from 70 to 100 *µ*m; 5% pores having a mean diameter in the range of from 101 to 115 *µ*m; 5% pores having a mean diameter in the range of from 116 to 130 *µ*m; 4% pores having a mean diameter in the range of from 131 to 300 *µ*m; 17% pores having a mean diameter in the range of from 301 to 330 *µ*m; 7% pores having a mean diameter in the range of from 331 to 360 *µ*m; 11% pores having a mean diameter in the range of from 361 to 390 *µ*m; 13% pores having a mean diameter in the range of from 391 to 420 *µ*m; 6% pores having a mean diameter in the range of from 421 to 450 *µ*m; 18% pores having a mean diameter in the range of from 451 to 480 *µ*m; and 11% pores having a mean diameter in the range of from 481 to 510 *µ*m.

5. The matrix as claimed in one of claims 1 to 4, **characterized in that** the biologically tolerated polymer is a biologically degradable polymer selected from natural polymers such as albumin, fibrinogen, collagen, gelatin, chitin, chitosan, agarose, alginate and synthetic polymers such as polyanhydrides, poly(ε-caprolactone) and poly(α-hydroxyesters).

6. The matrix as claimed in claim 5, **characterized in that** the biologically degradable polymer is poly(glycolic acid-lactic acid) having a lactic acid content of 85 mol% and a glycolic acid content of 15 mol%.

7. The porous matrix as claimed in one of claims 1 to 6, **characterized in that** the surface of the matrix is coated with at least one extracellular matrix protein selected from collagens, laminin and fibronectin.

8. The matrix as claimed in claim 7, **characterized in that** the coating contains a mixture of type I collagen, laminin and type IV collagen.

9. A method for preparing a porous matrix based on a biologically degradable polymer or polymer mixture according to any one of claims 1 to 8, **characterized in that** a mixture of polymer particles and a mixture of sodium chloride particles is compacted and the sodium chloride is then dissolved out, wherein
the sodium chloride particles consist of a mixture from 22 to 28% by weight of particles having a grain size of from 250 *µ*m to 320 *µ*m, from 42 to 46% by weight of particles having a grain size of from 330 to 380 *µ*m, and from 29 to 33% by weight of particles having a grain size of from 390 *µ*m to 425 *µ*m or
the sodium chloride particles consist of a mixture from 7 to 9% by weight of particles having a grain size of from 108 *µ*m to 140 *µ*m, from 5 to 7% by weight of particles having a grain size of from 145 *µ*m to 180 *µ*m, from 10 to 14% by weight of particles having a grain size of from 185 *µ*m to 220 *µ*m, from 5 to 7% by weight of particles having a grain size of from 225 *µ*m to 250 *µ*m, from 22 to 28% by weight of particles having a grain size of from 250 *µ*m to 320 *µ*m, from 22 to 28% by weight of particles having a grain size of from 330 *µ*m to 380 *µ*m, and from 15 to 19% by weight of particles having a grain size of from 390 *µ*m to 425 *µ*m, and
the polymer particles consist of a mixture from 14 to 18% by weight of particles having a grain size of from 108 *µ*m to 140 *µ*m, from 20 to 24% by weight of particles having a grain size of from 145 *µ*m to 180 *µ*m, from 43 to 49% by weight of particles having a grain size of from 185 *µ*m to 220 *µ*m, and from 14 to 18% by weight of particles having a grain size of from 225 *µ*m to 250 *µ*m.

10. The method as claimed in claim 9, **characterized in that** the ratio by weight of polymer particles to sodium chloride particles is from 1:100 to 1:10, preferably from 1:50 to 1:15 and in particular from 1:20 to 1:18.

11. The method as claimed in claim 9 or 10, **characterized in that** a polymer solution is added to the mixture composed of polymer particles and sodium chloride particles, and the solvent is removed, prior to the compacting.

12. The method as claimed in claim 11, **characterized in that** the solvent dissolves the polymer but not the salt.

13. The method as claimed in claim 12, **characterized in that** the solvent is selected from acetone, ethyl acetate, methylene chloride, chloroform, hexafluoroisopropanol, chlorinated and fluorinated, aliphatic and aromatic hydrocarbons, tetrahydrofuran, ethyl methyl ketone, diethyl ketone and mixtures thereof.

14. The method as claimed in claim 13, **characterized in that** the polymer is poly(glycolic acid), poly(lactic acid) or poly(glycolic acid-lactic acid) and the solvent is chloroform.

15. The method as claimed in any one of claims 11 to 14, **characterized in that** the ratio by weight of polymer particles to dissolved polymer is from 10:1 to 1:100, preferably from 2:1 to 1:25 and in particular from 1:1 to 1:10.

16. The method as claimed in claim 9, **characterized in that** the compacting is effected by the action of pressure.

17. The method as claimed in claim 9, **characterized in that** water is allowed to act on the compacted mixture for the purpose of dissolving out the sodium chloride.

18. The method as claimed in claim 17, **characterized in that** the water is removed once again.

19. The method as claimed in claim 9, **characterized in that** the compacted mixture is first of all stored in a CO₂ atmosphere and the sodium chloride is subsequently dissolved out.

20. The method as claimed in any one of claims 9 to 19, **characterized in that** a mixture composed of polymer particles, particles of sodium chloride and a polymer solution is compacted and the sodium chloride is then dissolved out.

21. The method as claimed in claim 20, **characterized in that** the polymer is selected from polyanhydrides, poly(orthoesters), poly(α-hydroxyesters), poly(ester amides), polyamides, poly(ester ethers), poly-carbonates, polyalkylenes, polyalkylene glycols, polyalkylene oxides, polyalkylene terephthalates, polyvinyl alcohols, polyvinyl ethers, polyvinyl esters, polyvinyl halides, polyvinylpyrrolidones, polysiloxanes, polystyrenes, polyurethanes, derivatized celluloses and (meth)acrylic acid polymers and copolymers.

22. The method as claimed in claim 20, **characterized in that** the solution contains polymer in dissolved form and polymer particles in solid form.

23. The method as claimed in claim 20, **characterized in that** the solution does not dissolve the sodium chloride.

24. An implant for tissue engineering which comprises a matrix as claimed in one of claims 1 to 8 and at least one cell.

25. The implant as claimed in claim 24 which comprises a matrix based on a biologically tolerated polymer and cells of at least two cell types, **characterized in that** the cells of the first cell type are hepatocytes and the cells of the second cell type are islet of Langerhans cells.

26. The implant as claimed in claim 25, **characterized in that** the ratio of hepatocytes to islet of Langerhans cells is 10⁶:3000.

27. The implant as claimed in claim 25, **characterized in that** the ratio of hepatocytes to islet of Langerhans cells is 10⁶:3-200, preferably 10⁶:10-100, in particular :20-80 and particularly preferably 10⁶:35-45.

## Revendications

1. Matrice poreuse à base d'un polymère ou d'un mélange de polymères biocompatible, **caractérisée en ce que** la matrice présente des pores d'une taille de 130 µm ou moins et des pores d'une taille de 370 µm ou plus, le degré de porosité est de 93 à 98% et la matrice peut être obtenue par compactage d'un mélange de particules polymères, présentant une grosseur de grain dans la plage de 20 à 950 µm, et de particules de chlorure de sodium, présentant une grosseur de grain dans la plage de 90 à 670 µm, et séparation par dissolution consécutive du chlorure de sodium, le degré de porosité étant l'indication numérique, en %, de la proportion du volume des pores par rapport au volume total de la matrice et la matrice présentant la répartition suivante des tailles de pore : 0,5% à 6% de pores présentant un diamètre moyen dans la plage de 70 à 100 µm ; 2% à 8% de pores présentant un diamètre moyen dans la plage de 101 à 115 µm ; 2% à 8% de pores présentant un diamètre moyen dans la plage de 116 à 130 µm ; 1% à 7% de pores présentant un diamètre moyen dans la plage de 131 à 300 µm; 11% à 23% de pores présentant un diamètre moyen dans la plage de 301 à 330 µm ; 4% à 10% de pores présentant un diamètre moyen dans la plage de 331 à 360 µm ; 5% à 17% de pores présentant un diamètre moyen dans la plage de 361 à 390 µm ; 7% à 19% de pores présentant un diamètre moyen dans la plage de 391 à 420 µm ; 3% à 9% de pores présentant un diamètre moyen dans la plage de 421 à 450 µm; 12% à 24% de pores présentant un diamètre moyen dans la plage de 451 à 480 µm ; et 5% à 17% de pores présentant un diamètre moyen dans la plage de 481 à 510 µm, les tailles de pore et la répartition des tailles de pore étant déterminées par microscopie électronique à balayage.

2. Matrice selon la revendication 1, **caractérisée en ce que** la matrice présente la répartition suivante des grosseurs de pore : 1% à 5% de pores présentant un diamètre moyen dans la plage de 70 à 100 µm ; 3% à 7% de pores présentant un diamètre moyen dans la plage de 101 à 115 µm ; 3% à 7% de pores présentant un diamètre moyen dans la plage de 116 à 130 µm ; 2% à 6% de pores présentant un diamètre moyen dans la plage de 131 à 300 µm; 13% à 21% de pores présentant un diamètre moyen dans la plage de 301 à 330 µm ; 5% à 9% de pores présentant un diamètre moyen dans la plage de 331 à 360 µm ; 7% à 15% de pores présentant un diamètre moyen dans la plage de 361 à 390 µm ; 9% à 17% de pores présentant un diamètre moyen dans la plage de 391 à 420 µm ; 4% à 8% de pores présentant un diamètre moyen dans la plage de 421 à 450 µm ; 14% à 22% de pores présentant un diamètre moyen dans la plage de 451 à 480 µm ; et 7% à 15% de pores présentant un diamètre moyen dans la plage de 481 à 510 µm.

3. Matrice selon la revendication 1, **caractérisée en ce que** la matrice présente la répartition suivante des grosseurs de pore : 2% à 4% de pores présentant un diamètre moyen dans la plage de 70 à 100 µm ; 4% à 6% de pores présentant un diamètre moyen dans la plage de 101 à 115 µm ; 4% à 6% de pores présentant un diamètre moyen dans la plage de 116 à 130 µm ; 3% à 5% de pores présentant un diamètre moyen dans la plage de 131 à 300 µm ; 15% à 19% de pores présentant un diamètre moyen dans la plage de 301 à 330 µm ; 6% à 8% de pores présentant un diamètre moyen dans la plage de 331 à 360 µm ; 9% à 13% de pores présentant un diamètre moyen dans la plage de 361 à 390 µm ; 11% à 15% de pores présentant un diamètre moyen dans la plage de 391 à 420 µm ; 5% à 7% de pores présentant un diamètre moyen dans la plage de 421 à 450 µm ; 16% à 20% de pores présentant un diamètre moyen dans la plage de 451 à 480 µm ; et 9% à 13% de pores présentant un diamètre moyen dans la plage de 481 à 510 µm.

4. Matrice selon la revendication 1, **caractérisée en ce que** la matrice présente la répartition suivante des grosseurs de pore : 3% de pores présentant un diamètre moyen dans la plage de 70 à 100 µm ; 5% de pores présentant un diamètre moyen dans la plage de 101 à 115 µm ; 5% de pores présentant un diamètre moyen dans la plage de 116 à 130 µm ; 4% de pores présentant un diamètre moyen dans la plage de 131 à 300 µm ; 17% de pores présentant un diamètre moyen dans la plage de 301 à 330 µm ; 7% de pores présentant un diamètre moyen dans la plage de 331 à 360 µm ; 11% de pores présentant un diamètre moyen dans la plage de 361 à 390 µm ; 13% de pores présentant un diamètre moyen dans la plage de 391 à 420 µm ; 6% de pores présentant un diamètre moyen dans la plage de 421 à 450 µm ; 18% de pores présentant un diamètre moyen dans la plage de 451 à 480 µm ; et 11% de pores présentant un diamètre moyen dans la plage de 481 à 510 µm.

5. Matrice selon l'une quelconque des revendications 1 à 4, **caractérisée en ce que** le polymère biocompatible est un polymère biodégradable qui est choisi parmi les polymères naturels, tels que l'albumine, le fibrinogène, le collagène, la gélatine, la quitine, le chitosane, l'agarose, l'alginate, et les polymères synthétiques, tels que les polyanhydrides, la poly(ε-caprolactone) et les poly(α-hydroxyesters).

6. Matrice selon la revendication 5, **caractérisée en ce que** le polymère biodégradable est le poly(acide glycolique-acide lactique) présentant une proportion d'acide lactique de 85% en mole et une proportion d'acide glycolique de 15% en mole.

7. Matrice selon l'une quelconque des revendications 1 à 6, **caractérisée en ce que** la surface de la matrice est revêtue par au moins une protéine de matrice extracellulaire, qui est choisie parmi les collagènes, la laminine et la fibronectine.

8. Matrice selon la revendication 7, **caractérisée en ce que** le revêtement contient de la fibronectine ou en ce que le revêtement contient un mélange de collagène de type I, de laminine et de collagène de type IV.

9. Procédé pour la préparation d'une matrice poreuse à base d'un polymère ou d'un mélange de polymères biocompatible selon l'une quelconque des revendications 1 à 8, **caractérisé en ce qu'**on compacte un mélange de particules polymères et de particules de chlorure de sodium et on sépare ensuite le chlorure de sodium par dissolution, les particules de chlorure de sodium étant constituées par un mélange de 22 à 28% en poids de particules présentant une grosseur de grain de 250 µm à 320 µm, de 42 à 46% en poids de particules présentant une grosseur de grain de 330 à 380 µm et de 29 à 33% en poids de particules présentant une grosseur de grain de 390 µm à 425 µm ou par un mélange de 7 à 9% en poids de particules présentant une grosseur de grain de 108 µm à 140 µm, de 5 à 7% en poids de particules présentant une grosseur de grain de 145 µm à 180 µm, de 10 à 14% en poids de particules présentant une grosseur de grain de 185 µm à 220 µm, de 5 à 7% en poids de particules présentant une grosseur de grain de 225 µm à 250 µm, de 22 à 28% en poids de particules présentant une grosseur de grain de 250 µm à 320 µm, de 22 à 28% en poids de particules présentant une grosseur de grain de 330 µm à 380 µm et de 15 à 19% en poids de particules présentant une grosseur de grain de 390 µm à 425 µm et les particules polymères étant constituées par un mélange de 14 à 18% en poids de particules présentant une grosseur de grain de 108 µm à 140 µm, de 20 à 24% en poids de particules présentant une grosseur de grain de 145 µm à 180 µm, de 43 à 49% en poids de particules présentant une grosseur de grain de 185 µm à 220 µm et de 14 à 18% en poids de particules présentant une grosseur de grain de 225 µm à 250 µm.

10. Procédé selon la revendication 9, **caractérisé en ce que** le rapport pondéral des particules polymères aux particules de chlorure de sodium est de 1:100 à 1:10, avantageusement de 1:50 à 1:15 et en particulier de 1:20 à 1:18.

11. Procédé selon la revendication 9 ou 10, **caractérisé en ce qu'**on ajoute au mélange de particules polymères et de particules de chlorure de sodium, avant le compactage, une solution de polymère et on élimine le solvant.

12. Procédé selon la revendication 11, **caractérisé en ce que** le solvant dissout le polymère mais pas le sel.

13. Procédé selon la revendication 12, **caractérisé en ce que** le solvant est choisi parmi l'acétone, l'acétate d'éthyle, le chlorure de méthylène, le chloroforme, l'hexafluoro-isopropanol, les hydrocarbures aliphatiques et aromatiques, chlorés et fluorés, le tétrahydrofuranne, l'éthylméthylcétone, la diéthylcétone ainsi que leurs mélanges.

14. Procédé selon la revendication 13, **caractérisé en ce que** le polymère est le poly(acide glycolique), le poly(acide lactique) ou le poly(acide glycolique-acide lactique) et le solvant est le chloroforme.

15. Procédé selon l'une quelconque des revendications 11 à 14, **caractérisé en ce que** le rapport pondéral des particules polymères au polymère dissous est de 10:1 à 1:100, avantageusement de 2:1 à 1:25 et en particulier de 1:1 à 1:10.

16. Procédé selon la revendication 9, **caractérisé en ce que** le compactage a lieu par l'action de la pression.

17. Procédé selon la revendication 9, **caractérisé en ce que**, pour la séparation par dissolution du chlorure de sodium, on laisse agir de l'eau sur le mélange compacté.

18. Procédé selon la revendication 17, **caractérisé en ce qu'**on élimine de nouveau l'eau.

19. Procédé selon la revendication 9, **caractérisé en ce que** le mélange compacté est d'abord entreposé dans une atmosphère de CO₂ et le chlorure de sodium est ensuite séparé par dissolution.

20. Procédé selon l'une quelconque des revendications 9 à 19, **caractérisé en ce qu'**on compacte un mélange de particules polymères, de particules de chlorure de sodium et d'une solution de polymère et on sépare ensuite le chlorure de sodium par dissolution.

21. Procédé selon la revendication 20, caractérisé le polymère est choisi parmi les polyanhydrides, les poly(orthoesters), les poly(α-hydroxyesters), les poly(esteramides), les polyamides, les poly(esteréthers), les polycarbonates, les polyalkylènes, les polyalkylèneglycols, les poly(oxydes d'alkylène), les poly(téréphtalates d'alkylène), les poly(alcools vinyliques), les polyvinyléthers, les poly(esters de vinyle), poly(halogénures de vinyle), les polyvinylpyrrolidones, les polysiloxanes, les polystyrènes, les polyuréthanes, les dérivés de cellulose et les polymères et copolymères de l'acide (méth)acrylique.

22. Procédé selon la revendication 20, **caractérisé en ce que** la solution contient le polymère sous forme dissoute et les particules polymères sous forme solide.

23. Procédé selon la revendication 20, **caractérisé en ce que** la solution ne dissout pas le chlorure de sodium.

24. Implant, comprenant une matrice selon l'une quelconque des revendications 1 à 8 et au moins une cellule.

25. Implant selon la revendication 24, comprenant une matrice à base d'un polymère biocompatible et des cellules d'au moins deux types de cellule, **caractérisé en ce que** les cellules du premier type de cellule sont des hépatocytes et les cellules du deuxième type de cellule sont des cellules d'îlots de Langerhans.

26. Implant selon la revendication 25, **caractérisé en ce que** le rapport des hépatocytes aux cellules d'îlots de Langerhans est de 10⁶:3000.

27. Implant selon la revendication 25, **caractérisé en ce que** le rapport des hépatocytes aux cellules d'îlots de Langerhans est de 10⁶:3-200, avantageusement de 10⁶:20-100, en particulier de :20-80 et de manière particulièrement préférée de 10⁶:35-45.
